# EUROPÄISCHE PATENTSCHRIFT

(11) **EP 0 543 346 B1**
(45) Veröffentlichungstag und Bekanntmachung des Hinweises auf die Patenterteilung: **07.09.1994**
(21) Anmeldenummer: 92119637.4
(22) Anmeldetag: 17.11.1992
(51) Int. Cl.: C07D 311/58, C07C 50/24

(54) **Verfahren zur Herstellung von substituierten Pentaalkylchromenen**
Method for the preparation of substituted pentaalkylchromenes
Procédé pour la préparation de pentaalkylchromenes substitués

(30) Priorität: 20.11.1991 CH 3392/91
(43) Veröffentlichungstag der Anmeldung: 26.05.1993
(73) Patentinhaber: LONZA AG, CH-3945 Gampel/Wallis (CH); Sankyo Company Limited, Tokyo (JP)
(72) Erfinder: Laffan, David, Dr., Visp (Kanton Wallis) (CH)
(74) Vertreter: Weinhold, Peter, Dr.

(56) Entgegenhaltungen:
- EP-A- 207 581
- EP-A- 0 036 169
- EP-A- 0 052 731
- GB-A- 2 055 097

## Beschreibung

Die Erfindung beinhaltet ein neues Verfahren zur Herstellung von Pentaalkylchromenen der allgemeinen Formel 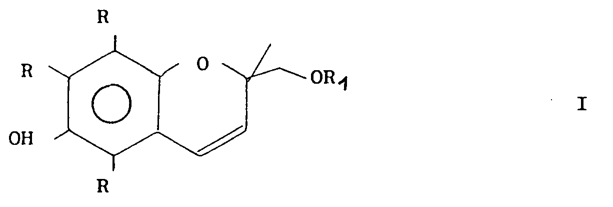
worin R eine Niederalkylgruppe mit 1 bis 4 C-Atomen und R₁eine Phenylgruppe unsubstituiert oder substituiert mit einer Niederalkylgruppe mit 1 bis 4 C-Atomen, mit Halogen oder mit einer Nitrogruppe,oder eine Alkanoylgruppe mit 1 bis 4 C-Atomen bedeutet sowie im Verfahren auftretende neue Zwischenprodukte.

Pentaalkylchromene, insbesondere die Tetramethylchromene sind wertvolle Zwischenprodukte zur Herstellung von z.B. lipidsenkenden Pharmazeutika (J.Med.Chem. 1989, 32, 421).

Aus der EP-PS 207 581 ist bekannt, entsprechende Pentaalkylchromene durch Kondensation von einem Acetylhydrochinon mit Alkoxy-/ oder Aryloxyacetonderivaten und durch Reduktion des resultierenden Chromanons herzustellen.

Diese Synthese hat den Nachteil, dass bereits die Ausgangsprodukte schwer zugänglich sind, ausserdem sind nach der Kondensation weitere Schritte notwendig, um zum gewünschten Pentaalkylchromen zu gelangen.
Es bestand daher die Aufgabe ein neues Verfahren zu entwickeln, das diese Nachteile nicht aufweist. Mit dem Verfahren gemäss Patentanspruch 1 konnte die Aufgabe gelöst werden.

Im ersten Schritt a) wird erfindungsgemäss ein Trialkylhydrochinon der allgemeinen Formel 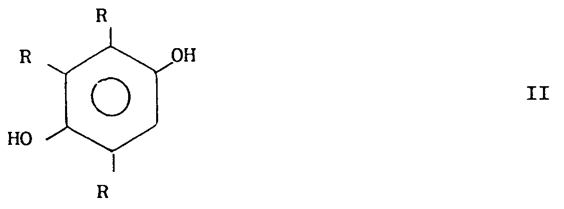
worin R die genannte Bedeutung hat,mit halogenierten Butenolen der Formel 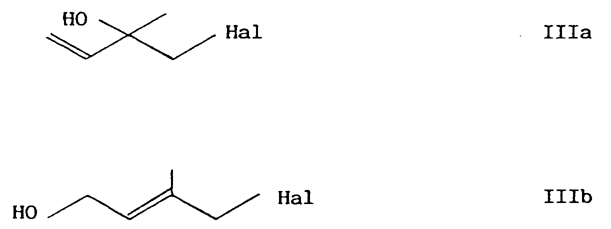
worin Hal Chlor, Brom oder Jod bedeutet, in Gegenwart einer Lewis-Säure so umgesetzt, dass als Zwischenprodukt ein Tetraalkylhydrochinon der allgemeinen Formel 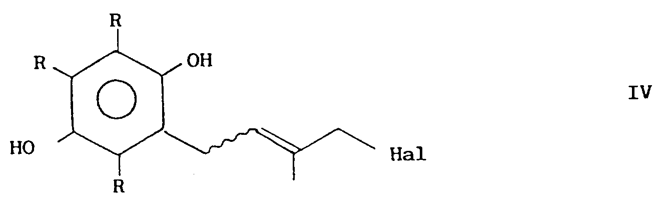
worin R und Hal die genannten Bedeutungen haben resultiert. Diese Verbindungen sind bisher nicht beschrieben.

Bevorzugt wird Trimethylhydrochinon (Formel II mit R = CH₃) mit 1-Chlor-2-methylbut-3-en-2-ol (Formel IIIa mit Hal = Cl) zum entsprechenden Tetraalkylhydrochinon (Formel IV mit R = CH₃ und Hal = Cl) umgesetzt.

Als Lewis-Säure wird zweckmässig Eisenchlorid, Zinntrifluormethansulfonat oder Bortrifluorid bzw. einer seiner Komplexe, bevorzugt Bortrifluorid oder eine seiner Komplexverbindungen, wie z.B. Bortrifluorid-etherat eingesetzt.

Die Lewis-Säure wird zweckmässig in einer Menge von 1 mol bis 5 mol, bezogen auf eingesetztes Trialkylhydrochinon eingesetzt.

Von Vorteil wird in Gegenwart eines inerten Lösungsmittels wie in Aromaten,z.B. in Toluol oder in halogenierten Kohlenwasserstoffen, z.B. in Methylenchlorid, bei einer Temperatur zwischen -10°C und 50°C, vorzugsweise bei Raumtemperatur gearbeitet.

Bevorzugt wird so verfahren, dass unmittelbar dann, wenn im Reaktionsgemisch praktisch kein Ausgangsprodukt (Trialkylhydrochinon) mehr nachweisbar ist, die Reaktion abgebrochen und das resultierende Tetraalkylhydrochinon isoliert wird.

Das Tetraalkylhydrochinon wird in der zweiten Stufe b) mit Luft oder Sauerstoff zum Chinon der allgemeinen Formel 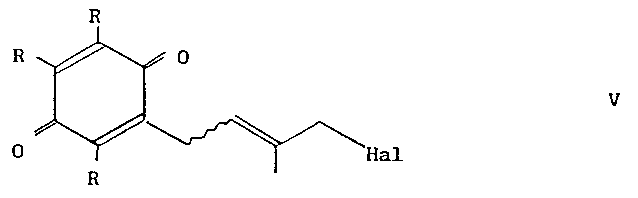
worin R und Hal die genannte Bedeutung haben, oxidiert.

Bevorzugt wird die Oxidation mit Sauerstoff durchgeführt.

Die Oxidation findet von Vorteil in Gegenwart eines inerten Lösungsmittels bei Raumtemperatur statt.
Üblicherweise ist die Umsetzung quantitativ.

Die resultierenden Chinone der allgemeinen Formel V sind bisher nicht beschrieben und daher ebenfalls Bestandteil der Erfindung. Bevorzugtes Chinon der allgemeinen Formel V ist das 2-(1-Chlor-2-methylbut-2-en-4-yl)-3,5,6-trimethyl-1,4-benzochinon mit R = CH₃ und Hal = Cl.

In der darauffolgenden 3. Stufe c) wird das Halogenatom im Chinon der allgemeinen Formel V mit einer nucleophilen Verbindung der allgemeinen Formel

(R₁-O)ₙ M VI

worin R₁ die genannte Bedeutung hat, M ein Alkali- oder Erdalkaliatom bedeutet und n 1 oder 2 ist nucleophil substituiert zu einem Chinon der allgemeinen Formel 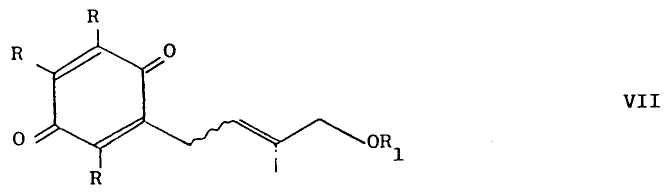
Geeignete nucleophile Verbindungen der allgemeinen Formel VI sind die Alkali- oder Erdalkalisalze der C₁-C₄-Carbonsäuren, bevorzugt der Essigsäure oder der substituierten Phenole, bevorzugt das p-Nitrophenol.
Abhängig von der Löslichkeit der Reaktionspartner wird die Reaktion in Gegenwart eines geeigneten Phasentransferkatalysators durchgeführt. Zweckmässig finden dazu Tetraalkylammoniumhalogenide oder Tetraalkylphosphoniumhalogenide wie z.B. das Tetrabutylammoniumjodid Anwendung.

Die Phasentransferkatalysatoren werden zweckmässig in einer Menge von 0,05 mol bis 1 mol, bezogen auf das eingesetzte Chinon der allgemeinen Formel V eingesetzt.

Die Umsetzung findet zweckmässig in Gegenwart eines polaren Losungsmittels, wie z.B. in Gegenwart von Acetonitril oder Methylenchlorid bei einer Temperatur zwischen 0°C und der Siedetemperatur (Rückflusstemperatur) des jeweiligen Lösungsmittels, vorzugsweise bei Rückflusstemperatur statt. Das resultierende nucleophil substituierte Chinon der allgemeinen Formel VII wird vorzugsweise nicht isoliert, sondern direkt entsprechend Stufe d) zum Endprodukt cyclisiert.

Die Cyclisierung kann entweder thermisch, säurekatalysiert oder basenkatalysiert erfolgen.
Zweckmässig wird säure- oder basenkatalysiert cyclisiert. Wahlt man die säurekatalysierte Cyclisierung, wird zweckmässig in Gegenwart einer Mineralsäure, wie z.B. Schwefelsäure, gearbeitet.
Bevorzugt wird die Cyclisierung basenkatalysiert mit einem Trialkylamin, wie z.B. Triethylamin,in einer Menge von 0,1 mol bis 2 mol, durchgeführt.

Zweckmässig wird in dem bereits für die Substitution verwendeten polaren Lösungsmittel, bevorzugt bei Rückflussbedingung gearbeitet.

Das resultierende Pentaalkylchromen der allgemeinen Formel I kann auf fachmännische Weise aus dem Reaktionsgemisch isoliert werden.

### Beispiel

### a) Verfahren zur Herstellung von 2-(1-Chlor-2-methylbut-2-en-4-yl)-1,4-dihydroxy-3,5,6-trimethylbenzol

Trimethylhydrochinon (2,00 g, 14 mmol) wurde in einer Mischung von Chlorbenzol (10 ml) und Hexan (5 ml) bei Raumtemperatur aufgeschlämmt. Bortrifluorid-diethylether-Komplex (3,60 ml einer 48%-igen Lösung von Bortrifluorid in Diethylether, (1,95 g Bortrifluorid, 28 mmol) wurde dann während 15 min zugegeben. Anschliessend wurde dann das 1-Chlor-2-methylbut-3-en-2-ol (2,42 g, 20 mmol) während 15 min zugetropft. Nach 30 min wurde die Reaktionsmischung filtriert, das Filtrat mit Chlorbenzol (10 ml) gewaschen und bei 40°C/20 mbar während 8 h getrocknet. Es wurden 2,24 g (62,9%) 2-(1-Chlor-2-methylbut-2-en-4-yl)-1,4-dihydroxy-3,5,6-trimethylbenzol erhalten. Schmelzpunkt: 118-122°C (Zersetzung)
- ¹H-NMR: (CDCl₃, 300 MHz) δ in ppm:: 5,50 (t, 1H, J=7,5Hz);
4,02 (s, 2H);
3,45 (d, 2H, J=7,5Hz);
2,17 (s, 6H);
2,15 (s, 3H);
1,95 (s, 3H)
- Isomer:: 5,48 (t, 1H, J=7,5Hz);
4,02 (s, 2H);
3,48 (d, 2H, J=7,5Hz);
2,17 (s, 6H);
2,15 (s, 3H);
1,95 (s, 3H)

### b) Verfahren zur Herstellung von 2-(1-Chlor-2-methylbut-2-en-4-yl)-3,5,6-trimethyl-1,4-benzochinon

2-(1-Chlor-2-methylbut-2-en-4-yl)-1,4-dihydroxy-3,5,6-trimethylbenzol (5,6 g, 22,0 mmol) wurden in Chloroform (100 ml) bei Raumtemperatur aufgeschlämmt. Sauerstoff (O₂ (g)) wurde eingeleitet. Nach 4 h wurde die Sauerstoff-Zugabe abgebrochen, die Reaktionsmischung abfiltriert und die Mutterlauge eingeengt. Dies ergab 2-(1-Chlor-2-methylbut-2-en-4-yl)-3,5,6-trimethyl-1,4-benzochinon (5,6 g, 99%).
- ¹H-NMR: (CDCl₃, 300 MHz) δ in ppm:: 5,38 (t, 1H, J=7,5Hz);
3,99 (s, 2H);
3,26 (d, 2H, J=7,5Hz);
2,05 (s, 3H);
2,02 (s, 6H);
1,90 (s, 3H)
- Isomer:: 5,25 (t, 1H, J=7,5Hz);
4,24 (s, 2H);
3,29 (d, 2H, J=7,5Hz);
2,08 (s, 3H);
2,02 (s, 6H);
1,90 (s, 3H)

### c) Verfahren zur Herstellung von 6-Hydroxy-2-(4-nitrophenoxymethyl)-2,5,7,8-tetramethylchrom-3-en

2-(1-Chlor-2-methylbut-2-en-4-yl)-3,5,6-trimethyl-1,4-benzochinon (2,53 g, 10 mmol) wurden in Acetonitril, mit Natrium p-nitrophenolat (1,95 g, Gehalt 82,7%, 10 mmol) und Tetrabutylammoniumjodid (0,37 g, 1 mmol) versetzt und zum Rückfluss erwärmt. Nach 1 h wurde Triethylamin (1,0 g, 10 mmol) zugegeben.
Nach 5 h wurde das Gemisch auf Raumtemperatur gekühlt und dann abgenutscht. Das 6-Hydroxy-2-(4-nitrophenoxyphenyl)-2,5,7,8-tetramethylchrom-3-en wurde chromatographisch isoliert.
- ¹H-NMR: (CDCl₃, 300 MHz) δ in ppm:: 8,15 (d, 2H, J=8Hz);
6,95 (d, 2H, J=8Hz);
6,70 (d, 1H, J=9Hz);
5,72 (d, 1H, J=9Hz);
4,28 (s, 1H);
4,11 (d, 1H, J=10Hz);
4,03 (d, 1H, J=10Hz);
2,21 (s, 3H);
2,15 (s, 3H);
2,06 (s, 3H);
1,56 (s, 3H)

## Patentansprüche

1. Verfahren zur Herstellung von subst. Pentaalkylchromenen der allgemeinen Formel 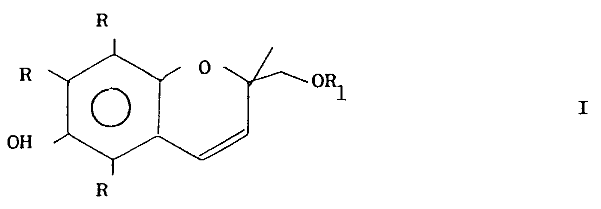 worin R eine Niederalkylgruppe mit 1 bis 4 C-Atomen und R₁ eine Phenylgruppe unsubstituiert oder substituiert mit einer Niederalkylgruppe mit 1 bis 4 C-Atomen, mit Halogen oder mit einer Nitrogruppe, oder eine Alkanoylgruppe mit 1 bis 4 C-Atomen bedeutet, dadurch gekennzeichnet, dass ein Trialkylhydrochinon der allgemeinen Formel 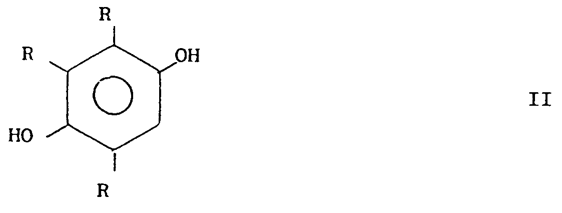 worin R die genannte Bedeutung hat, in der ersten Stufe a) mit einem Butenol der Formel 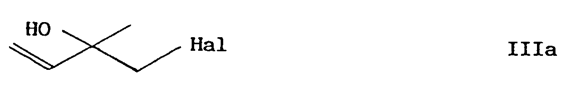 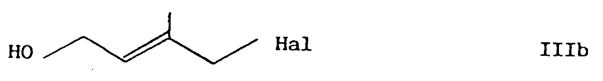 worin Hal Chlor, Brom oder Jod bedeuten, in Gegenwart einer Lewis-Säure zu einem Tetraalkylhydrochinon der allgemeinen Formel 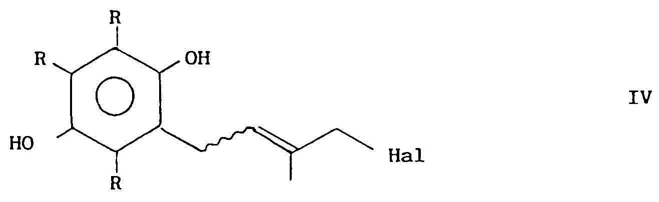 worin R und Hal die angegebene Bedeutung haben, umgesetzt wird, b) in der zweiten Stufe das Tetraalkylhydrochinon mit Luft oder Sauerstoff zum Chinon der allgemeinen Formel 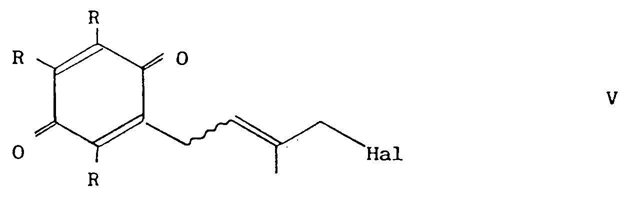 worin R und Hal die genannte Bedeutung besitzen, oxidiert wird, c) in der dritten Stufe das Halogenatom mit einer nucleophilen Verbindung der allgemeinen Formel
(R₁-O)ₙ M VI
worin R₁ die genannte Bedeutung hat und M ein Alkali- oder Erdalkalimetallatom bedeutet und n 1 oder 2 ist, nucleophil substituiert wird zu einem Chinon der allgemeinen Formel 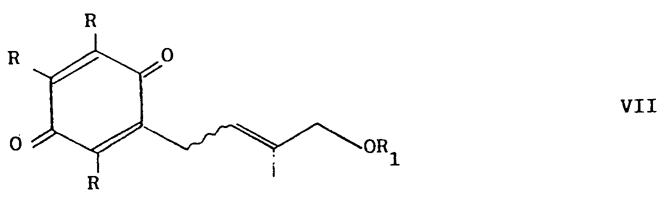 worin R und R₁ die genannte Bedeutung haben, und d) zum Endprodukt cyclisiert wird.

2. Verfahren nach Patentanspruch 1, dadurch gekennzeichnet, dass als Trialkylhydrochinon der allgemeinen Formel II das Trimethylderivat mit R = CH₃ und als halogeniertes Butenolderivat das 1-Chlor-2-methylbut-3-en-2-ol der Formel IIIa mit Hal = Cl eingesetzt wird.

3. Verfahren nach Patentanspruch 1 oder 2, dadurch gekennzeichnet, dass als Lewis-Säure Bortrifluorid oder eine seiner Komplexverbindungen eingesetzt wird.

4. Verfahren nach einem der Patentansprüche 1 bis 3, dadurch gekennzeichnet, dass die Umsetzung zum Tetraalkylhydrochinon bei einer Temperatur zwischen -10°C und 50°C in Gegenwart eines inerten Lösungsmittels durchgeführt wird.

5. Verfahren nach einem der Patentansprüche 1 bis 4, dadurch gekennzeichnet, dass die Umsetzung zum Tetraalkylhydrochinon so durchgeführt wird, dass unmittelbar dann, wenn im Reaktionsgemisch das Ausgangsprodukt Trialkylhydrochinon praktisch nicht mehr nachweisbar ist, die Reaktion abgebrochen und das resultierende Tetraalkylhydrochinon isoliert wird.

6. Verfahren nach einem der Patentansprüche 1 bis 5, dadurch gekennzeichnet, dass die Oxidation mit Sauerstoff bei Raumtemperatur in Gegenwart eines inerten Lösungsmittels erfolgt.

7. Verfahren nach einem der Patentansprüche 1 bis 6, dadurch gekennzeichnet, dass die nucleophile Substitution in der Stufe c) in Gegenwart eines Phasentransferkatalysators durchgeführt wird.

8. Verfahren nach einem der Patentansprüche 1 bis 7, dadurch gekennzeichnet, dass die nucleophile Substitution in der Stufe c) in einem polaren Lösungsmittel bei einer Temperatur zwischen 0°C und der Siedetemperatur des Lösungsmittels durchgeführt wird.

9. Verfahren nach einem der Patentansprüche 1 bis 8, dadurch gekennzeichnet, dass als nucleophile Verbindung der Formel VI ein Alkalisalz des p-Nitrophenols eingesetzt wird.

10. Verfahren nach einem der Patentansprüche 1 bis 9, dadurch gekennzeichnet, dass die Cyclisierung in Stufe d) thermisch-, säurekatalysiert oder basenkatalysiert durchgeführt wird.

11. Verfahren nach Patentanspruch 10, dadurch gekennzeichnet, dass die Cyclisierung basenkatalytisch mit einem Trialkylamin durchgeführt wird.

12. Verfahren nach einem der Patentansprüche 1 bis 11, dadurch gekennzeichnet, dass die nucleophile Substitution in Stufe c) und die Cyclisierung in Stufe d) ohne Isolierung des Chinons der allgemeinen Formel VII durchgeführt wird.

13. Chinone der allgemeinen Formel 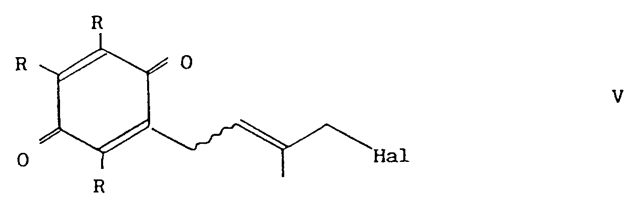 worin R und Hal die genannte Bedeutung haben.

14. 2-(1-Chlor-2-methylbut-2-en-4-yl)-3,5,6-trimethyl-1,4-benzochinon der Formel

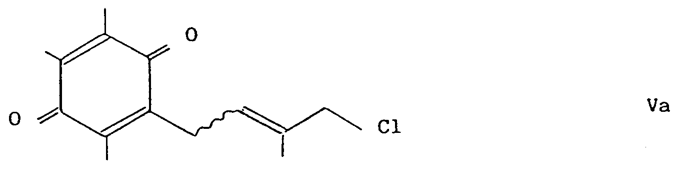

## Claims

1. A process for the preparation of substituted pentaalkyl chromenes of the general formula: 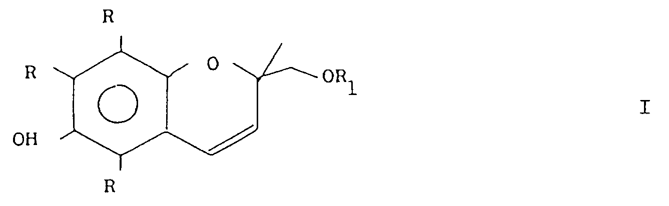 wherein R is a lower alkyl group having 1 to 4 carbon atoms, and R₁ is a phenyl group either unsubstituted or substituted with a lower alkyl group having 1 to 4 carbon atoms, with halogen or with a nitro group, or an alkanoyl group having 1 to 4 carbon atoms;
characterized by the steps of:
(a) reacting a trialkyl hydroquinone of the general formula: 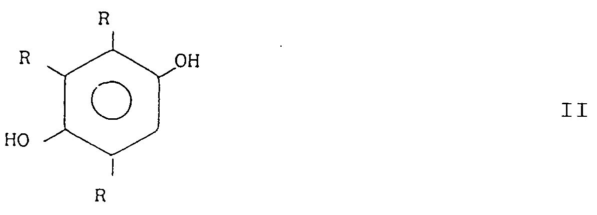 wherein R has the above meaning; in a first step with a butenol of the formula: 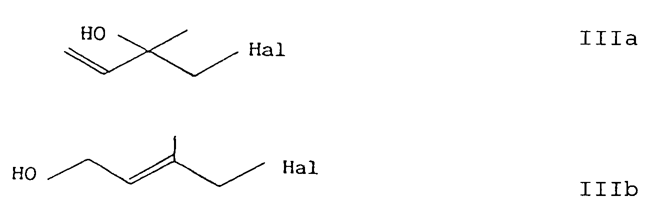 wherein Hal is chlorine, bromine or iodine; in the presence of a Lewis acid to form a tetraalkyl hydroquinone of the general formula: 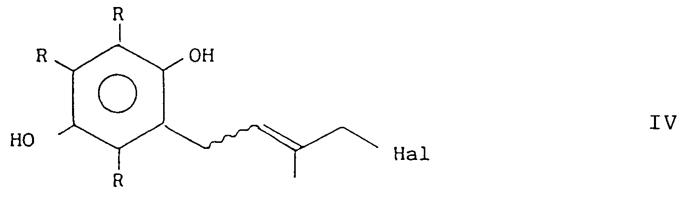 wherein R and Hal have the above meaning; (b) oxidizing in a second step the tetraalkyl hydroquinone with air or oxygen to form a quinone of the general formula: 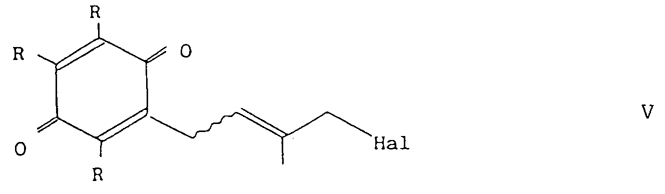 wherein R and Hal have the above meaning; (c) nucleophilically substituting in a third step the halogen atom with a nucleophilic compound of the general formula:
(R₁-O)ₙ M VI
wherein R₁ has the above meaning, and M is an alkali or alkaline earth metal atom, and n is 1 or 2; to form a guinone of the general formula: 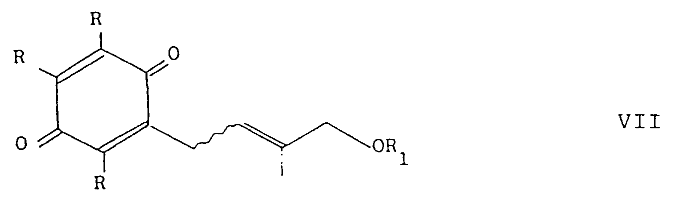 wherein R and R₁ have the above meaning; which is (d) cyclized to the final product.

2. The process according to Claim 1, characterized in that a trimethyl derivative wherein R = CH₃ is used as the trialkyl hydroquinone of general formula II, and a 1-chloro-2-methylbut-3-en-2-ol of formula IIIa wherein Hal = Cl is used as a halogenated butenol derivative.

3. The process according to Claim 1 or 2, characterized in that boron trifluoride or one of its complex compounds is used as a Lewis acid.

4. The process according to any one of Claims 1 to 3, characterized in that reaction to form the tetraalkyl hydroquinone is conducted at a temperature between -10°C and 50°C in the presence of an inert solvent.

5. The process according to any one of Claims 1 to 4, characterized in that reaction to form tetraalkyl hydroquinone is conducted in such a manner that, as soon as the starting trialkyl hydroquinone is practically no longer detectable in the reaction mixture, reaction is stopped, and the resulting tetraalkyl hydroquinone is isolated.

6. The process according to any one of Claims 1 to 5, characterized in that oxidation with oxygen takes place at room temperature in the presence of an inert solvent.

7. The process according to any one of Claims 1 to 6, characterized in that nucleophilic substitution in step (c) is conducted in the presence of a phase transfer catalyst.

8. The process according to any one of Claims 1 to 7, characterized in that nucleophilic substitution in step (c) takes place in a polar solvent at a temperature between 0°C and the boiling temperature of the solvent.

9. The process according to any one of Claims 1 to 8, characterized in that an alkali salt of p-nitrophenol is used as the nucleophilic compound.

10. The process according to any one of Claims 1 to 9, characterized in that cyclization in step (d) is conducted either thermally, or acid-catalyzed or base-catalyzed.

11. The process according to Claim 10, characterized in that cyclization is base-catalyzed using a trialkyl amine.

12. The process according to any one of Claims 1 to 11, characterized in that nucleophilic substitution in step (c) and cyclization step (d) are conducted without isolating the quinone of general formula VII.

13. Quinones of the general formula: 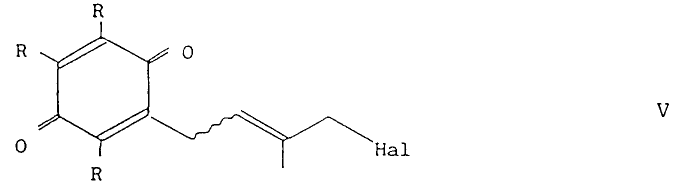 wherein R and Hal have the above meaning.

14. 2-(1-Chloro-2-methylbut-2-en-4-yl)-3,5,6-trimethyl-1,4-benzoquinone of the formula:

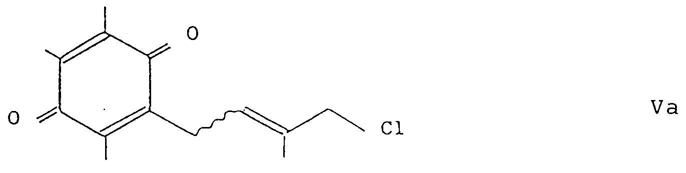

## Revendications

1. Procédé pour la préparation de pentaalkylchromènes substitués de la formule générale 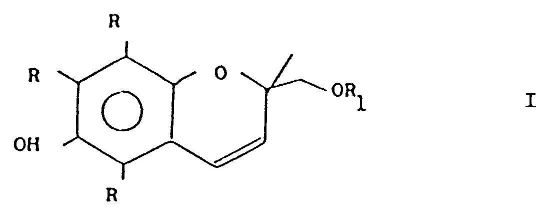 dans laquelle R est un groupe alkyle inférieur avec 1 jusqu'à 4 atomes C et R₁ signifie un groupe phényle non substitué ou substitué avec un groupe alkyle inférieur ayant 1 à 4 atomes C, avec l'halogène ou avec un groupe nitro ou un groupe alcanoyle ayant 1 jusqu'à 4 atomes C, caractérisé en ce que l'on met en réaction une trialkylhydroquinone de la formule générale 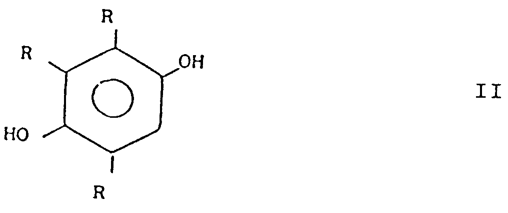 dans laquelle R a la signification indiquée, dans la première étape a) avec un buténol de la formule 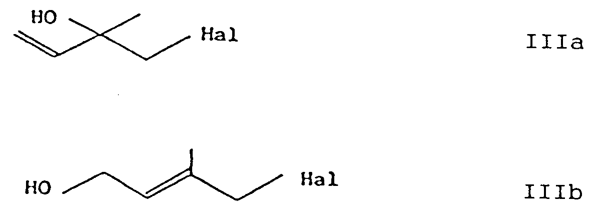 dans laquelle Hal signifie chlore, brome ou iode, en présence d'un acide Lewis pour donner une tétraalkylhydroquinone de la formule générale 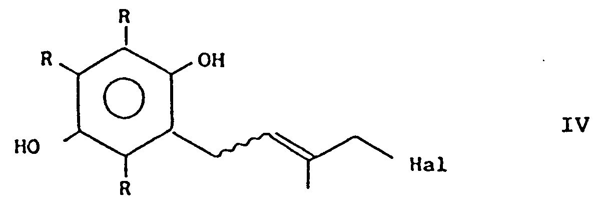 dans laquelle R et Hal ont la signification indiquée, b) dans la deuxième étape, on oxyde la tétraalkylhydroquinone avec de l'air ou de l'oxygène pour donner de la quinone de formule générale 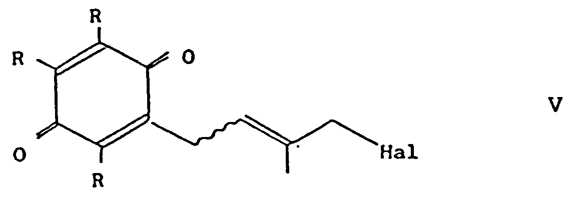 dans laquelle R et Hal ont la signification indiquée, c) dans la troisième étape, l'atome halogène est substitué par un composé nucléophile de la formule générale
(R₁-O)ₙ M VI
dans laquelle R₁ a la signification indiquée, M signifie un atome des métaux alcalins ou alcalino-terreux et n est égal à 1 ou à 2, substitué par nucléophile pour donner une quinone de la formule générale 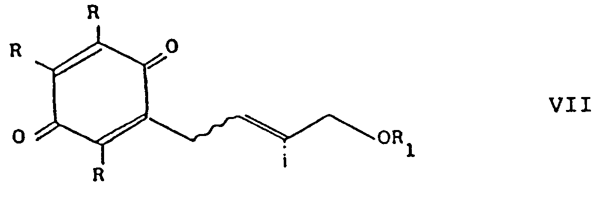 dans laquelle R et R₁ ont la signification indiquée et d) est soumis à cyclisation pour donner le produit final.

2. Procédé selon la revendication 1, caractérisé en ce qu'en tant que trialkylhydroquinone de la formule générale II, on met en oeuvre le dérivé de triméthyle avec R = CH₃ et comme dérivé de buténol halogéné, on met en oeuvre le 1-chlor-2-méthylbut-3-en-2-ol de la formule IIIa avec Hal = Cl.

3. Procédé selon la revendication 1 ou 2, caractérisé en ce qu'en tant qu'acide de Lewis, on met en oeuvre du trifluorure de bore ou l'une des ses compositions complexes.

4. Procédé selon l'une des revendications 1 à 3, caractérisé en ce que la réaction en tétraalkylhydroquinone est conduite à une température entre -10°C et 50°C en présence d'un solvant inerte.

5. Procédé selon l'une des revendications 1 à 4, caractérisé en ce que la réaction en tétraalkylhydroquinone est conduite de façon que dès qu'il n'y a pratiquement plus de produit de départ trialkylhydroquinone dans le mélange réactionnel, on interrompt la réaction et la tétraalkylhydroquinone résultante est isolée.

6. Procédé selon l'une des revendications 1 à 5, caractérisé en ce que l'oxydation s'effectue avec de l'oxygène à température ambiante en présence d'un solvant inerte.

7. Procédé selon l'une des revendications 1 à 6, caractérisé en ce que la substitution nucléophile dans l'étape c) s'effectue en présence d'un catalyseur de transfert de phase.

8. Procédé selon l'une des revendications 1 à 7, caractérisé en ce que la substitution nucléophile dans l'étape c) est conduite dans un solvant polaire à une température entre 0°C et la température d'ébullition du solvant.

9. Procédé selon l'une des revendications 1 à 8, caractérisé en ce qu'en tant que composé nucléophile de formule VI, on met en oeuvre un sel alcalin de p-nitrophénol.

10. Procédé selon l'une des revendications 1 à 9, caractérisé en ce que la cyclisation est effectuée à l'étape d) thermiquement, par catalyse acide ou par catalyse de base.

11. Procédé selon la revendication 10, caractérisé en ce que la cyclisation est conduite par catalyse de base avec une trialkylamine.

12. Procédé selon l'une des revendications 1 à 11, caractérisé en ce que la substitution nucléophile à l'étape c) et la cyclisation à l'étape d) s'effectuent sans isoler la quinone de formule générale VII.

13. Quinone de formule générale 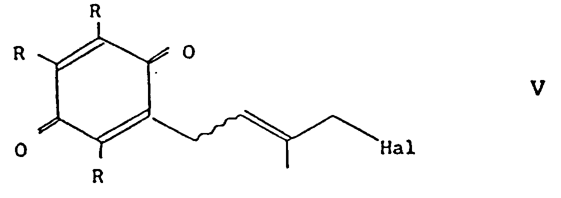 dans laquelle R et Hal ont la signification indiquée.

14. 2-(1-chlor-2-méthylbut-2-en-4-yl)-3,5,6-triméthyl-1,4-benzoquinone de la formule
